# EUROPEAN PATENT APPLICATION

(11) **EP 1 688 148 A1**
(43) Date of publication of application: **09.08.2006**
(21) Application number: 06112986.2
(22) Date of filing: 01.12.2003
(51) Int. Cl.: A61K 38/22, A61K 31/426, A61K 31/427, A61P 3/10

(54) **Combination treatment using exendin-4 and thiazolidinediones**

(30) Priority: 03.12.2002 DK 200201864; 09.12.2002 US 431999 P
(62) Divisional of application: 03775117.9
(71) Applicant: NOVO NORDISK A/S, 2880 Bagsvaerd (DK)
(72) Inventor: The designation of the inventor has not yet been filed
(74) Representative: Jensen, Dorte Kold

(57) **Abstract**

The present invention relates to methods for treatment and/or prevention of diabetes and diabetes related diseases. More specifically, the methods and uses of the invention pertains to administration of an exendin-4 compound in combination with administration of a thiazolidinedione insulin sensitizer.

## Description

### FIELD OF THE INVENTION

The present invention relates to combination treatment of patients using an exendin peptide and a thiazolidinedione. More specifically, the methods and uses of the invention pertains to administration of an exendin-4 peptide in combination with administration of a thiazolidinedione insulin sensitizer.

### BACKGROUND OF THE INVENTION

Diabetes is a disorder of carbohydrate metabolism characterized by hyperglycemia and glucosuria resulting from insufficient production or utilization of insulin. Diabetes severely affects the quality of life of large parts of the populations in developed countries. Insufficient production of insulin is characterised as type 1 diabetes and insufficient utilization of insulin is type 2 diabetes. However, it is now widely recognised that there are many distinct diabetes related diseases which have their onset long before patients are diagnosed as having overt diabetes. Also, the effects from the suboptimal control of glucose metabolism in diabetes gives rise to a wide spectrum of related lipid and cardiovascular disorders.

Dyslipidemia, or abnormal levels of lipoproteins in blood plasma, is a frequent occurrence among diabetics. Dyslipidemia is typically characterized by elevated plasma triglycerides, low HDL (High Density Lipoprotein) cholesterol, normal to elevated levels of LDL (Low Density Lipoprotein) cholesterol and increased levels of small dense, LDL (Low Density Lipoprotein) particles in the blood. Dyslipidemia is one of the main contributors to the increased incidence of coronary events and deaths among diabetic subjects. Epidemiological studies have confirmed this by showing a several-fold increase in coronary deaths among diabetic subjects when compared with non-diabetic subjects. Several lipoprotein abnormalities have been described among diabetic subjects.

Insulin resistance is the diminished ability of insulin to exert its biologically action across a broad range of concentrations. In insulin resistance, the body secretes abnormally high amounts of insulin to compensate for this defect and a state of impaired glucose tolerance develops. Failing to compensate for the defective insulin action, the plasma glucose concentration inevitable rises, resulting in the clinical state of diabetes. It is being recognised that insulin resistance and relative hyperinsulinemia have a contributory role in obesity, hypertension, atherosclerosis and type 2 diabetes. The association of insulin resistance with obesity, hypertension and angina has been described as a syndrome, Syndrome X, having insulin resistance as the common pathogenic link.

Apoptosis is an active process of cellular self-destruction that is regulated by extrinsic and intrinsic signals occurring during normal development. It is well documented that apoptosis plays a key role in regulation of pancreatic endocrine beta cells. There is increasing evidence that in adult mammals the beta-cell mass is subject to dynamic changes to adapt insulin production for maintaining euglycemia in particular conditions, such as pregnancy and obesity. The control of beta cell mass depends on a subtle balance between cell proliferation, growth and programmed cell death (apoptosis). A disturbance of this balance may lead to impairment of glucose homeostasis. For example, it is noteworthy that glucose intolerance develops with aging when beta cell replication rates are reduced and human autopsy studies repeatedly showed a 40-60% reduction of beta cell mass in patients with non-insulin-dependent-diabetes mellitus compared with nondiabetic subjects. It is generally agreed that insulin resistance is an invariable accompaniment of obesity but that normoglycemia is maintained by compensatory hyperinsulinemia until the beta cells become unable to meet the increased demand for insulin, at which point type 2 diabetes begins.

Attempts to treatment of the multiple abnormalities associated with diabetes have prompted for the administration of several anti-diabetic medicaments in order to address these abnormalities in the different patients. Examples of anti-diabetic medicaments are proteins such as insulin and insulin analogues, and small molecules such as insulin sensitizers, insulin secretagogues and appetite regulating compounds.

In the last decade a number of peptides have been isolated from the venom of the Gila monster lizards *(Heloderma suspectum* and *Heloderma horridum).* Exendin-4 is a 39 amino acid residue peptide isolated from the venom of *Heloderma suspectum,* and this peptide shares 52% homology with GLP-1, the human insulinotropic hormone. Exendin-4 is a potent GLP-1 receptor agonist which has been shown to stimulate insulin release and ensuing lowering of the blood glucose level when injected into dogs. The group of exendin-4(1-39), certain fragments thereof, analogs thereof and derivatives thereof, are potent insulinotropic agents. Most importantly the group of exendin-4(1-39), insulinotropic fragments thereof, insulinotropic analogs thereof and insulinotropic derivatives thereof. Side effects observed with exendins are nausea and a quite dramatic immunological response have been reported.

PCT publication WO 00/78333 describes co-administration of GLP-1 and thiazolidinedione for treatment of NIDDM. A side effect of thiazolidinedione was stated to be reduced and a synergistic effect of combining a GLP-1 analogue with a thiazolidinedione is claimed.

Combined treatment with exendins and thiazolidinediones convey the benefits of both compounds while reducing side effects which are associated with each compound when used at the optimal dosage for single compound therapy. Thus, there is a need for the therapeutic benefits of the individual compounds while simultaneously reducing the side effects.

### SUMMARY OF THE INVENTION

One object of the present invention is to provide methods, which can effectively be used in the treatment of diabetes and diabetes related diseases selected from the group consisting of type 1 diabetes, type 2 diabetes, hyperglycemia, type 1.5 diabetes, latent autoimmune diabetes in adults, maturity onset diabetes, beta-cell apoptosis, hemochromatosis induced diabetes, impaired glucose tolerance, metabolic syndrome X, insulin resistance, cystic fibrosis related diabetes, polycystic ovarian syndrome, gestational diabetes, obesity, dyslipidemia, diabetic dyslipidemia, hyperlipidemia, hypertriglyceridemia, hyperlipoproteinemia, hypercholesterolemia, hypertension, essential hypertension, acute hypertensive emergency, arteriosclerosis, atherosclerosis, intermittent claudication (atherosclerosis oblitterens), cardiovascular disease, cardiomyopathy, cardiac hypertrophy, left ventricular hypertrophy, coronary artery disease, early coronary artery disease, heart insufficiency, exercise tolerance, chronic heart failure, mild chronic heart failure, arrhythmia, cardiac dysrythmia, syncopy, heart attack, myocardial infarction, Q-wave myocardial infarction, stroke, acute coronary syndrome, angina pectoris, unstable angina, cardiac bypass reocclusion, diastolic dysfunction, systolic dysfunction, non-Q-wave cardiac necrosis, catabolic changes after surgery, acute pancreatitis, irritable bowel syndrome, diabetic retinopathy, background retinopathy, preproliferative retinopathy, proliferative retinopathy, macular edema, cataracts, nephropathy, diabetic nephropathy, microalbuminuria, macroalbuminuria, neuropathy, diabetic neuropathy, distal symmetrical sensorimotor polyneuropathy, and diabetic autonomic neuropathy, which methods comprise administration of an effective amount of an exendin-4 compound and administration of an effective amount of a thiazolidinedione insulin sensitizer to a patient in need thereof.

Another object of the invention is to provide methods for increasing the number of beta-cells in a patient, increasing the size of beta-cells in a patient or stimulating beta-cell proliferation in a patient in need thereof, which method comprises administration of an effective amount of an exendin-4 compound and an effective amount of a thiazolidinedione insulin sensitizer to a patient in need thereof.

The two compounds, i.e. the exendin-4 compound and the thiazolidinedione insulin sensitizer, may be co-administered or they may be administered separately as two medicaments. Furthermore, the first compound may be administered in a regimen, which additionally comprises treatment with the second compound.

Another object of the invention is to provide novel pharmaceutical compositions comprising an exendin-4 peptide and a thiazolidinedione insulin sensitizer.

In one embodiment of the invention, the exendin-4 compound is exendin-4(1-39) and the thiazolidinedione insulin sensitizer is troglitazone, pioglitazone, 5-[[4-[3-methyl-4-oxo-3,4-dihydro-2-quinazolinyl]methoxy]phenyl-methyl]thiazolidine-2,4-dione, or a salt thereof

In yet another embodiment of the invention the thiazolidinedione insulin sensitizer and the exendin-4 compound are administered in suboptimal dosages.

In yet another embodiment of the invention the thiazolidinedione insulin sensitizer and the exendin-4 compound are administered in amounts and for a sufficient time to produce a synergistic effect.

### DEFINITIONS

The following is a detailed definition of the terms used in the specification.

The term "co-administration" as used herein means administration of the two compounds to the patient within a period of one month. The term includes separate administration of two medicaments each containing one of the compounds as well as simultaneous administration whether or not the two compounds are combined in one formulation or whether they are in two separate formulations.

The term "effective amount" as used herein means a dosage which is sufficient in order for the treatment of the patient to be effective compared with no treatment.

The term "medicament" as used herein means a pharmaceutical composition suitable for administration of the pharmaceutically active compound to a patient.

The term "suboptimal dosage" us used herein means a dosage which is below the optimal dosage for that compound when used in single-compound therapy.

The term "additive effect" as used herein means the effect resulting from the sum of the effects obtained from the individual compounds.

The term "synergistic effect" as used herein means an effect which is greater than the additive effect which results from the sum of the effects of the two individual compounds.

The term "treatment of a disease" as used herein means the management and care of a patient having developed the disease, condition or disorder. The purpose of treatment is to combat the disease, condition or disorder. Treatment includes the administration of the active compounds to eliminate or control the disease, condition or disorder as well as to alleviate the symptoms or complications associated with the disease, condition or disorder.

The term "prevention of a disease" as used herein is defined as the management and care of an individual at risk of developing the disease prior to the clinical onset of the disease. The purpose of prevention is to combat the development of the disease, condition or disorder, and includes the administration of the active compounds to prevent or delay the onset of the symptoms or complications and to prevent or delay the development of related diseases, conditions or disorders.

The term "exendin-4 compound" as used herein is defined as exendin-4(1-39) (SEQ ID No. 1 or the C-terminally amidated version thereof), insulinotropic fragments thereof, insulinotropic analogs thereof and insulinotropic derivatives thereof. Insulinotropic fragments of exendin-4 are insulinotropic peptides for which the entire sequence can be found in the sequence of exendin-4 (SEQ ID No. 1) and where at least one terminal amino acid has been deleted. Examples of insulinotropic fragments of exendin-4(1-39) are exendin-4(1-38) and exendin-4(1-31). The insulinotropic property of a compound may be determined by in vivo or in vitro assays well known in the art. For instance, the compound may be administered to an animal and monitoring the insulin concentration over time. Insulinotropic analogs of exendin-4(1-39) refer to the respective molecules wherein one or more of the amino acids residues have been exchanged with other amino acid residues and/or from which one or more amino acid residues have been deleted and/or from which one or more amino acid residues have been added with the proviso that said analogue either is insulinotropic or is a prodrug of an insulinotropic compound . An example of an insulinotropic analog of exendin-4(1-39) is Ser²Asp³-exendin-4(1-39) wherein the amino acid residues in position 2 and 3 have been replaced with serine and aspartic acid, respectively (this particular analog also being known in the art as exendin-3). Insulinotropic derivatives of exendin-4(1-39) and analogs thereof are what the person skilled in the art considers to be derivatives of these peptides, i.e. having at least one substituent which is not present in the parent peptide molecule with the proviso that said derivative either is insulinotropic or is a prodrug of an insulinotropic compound. Examples of substituents are amides, carbohydrates, alkyl groups, esters and lipophilic substituents. An example of an insulinotropic derivatives of exendin-4(1-39) and analogs thereof is Tyr³¹-exendin-4(1-31)-amide.

The term "prodrug of an insulinotropic compound" as used herein means a chemically modified compound which following administration to the patient is converted to an insulinotropic compound. Such prodrugs are typically amino acid extended versions or esters of the insulinotropic compound.

The term "stable exendin-4 compound" as used herein means a chemically modified exendin-4(1-39), i.e. an analogue, a derivative, or a fusion protein, which exhibits an in vivo plasma elimination half-life of at least 10 hours in man, as determined by the following method. The method for determination of plasma elimination half-life of an exendin-4 compound in man is : The compound is dissolved in an isotonic buffer, pH 7.4, PBS or any other suitable buffer. The dose is injected peripherally, preferably in the abdominal or upper thigh. Blood samples for determination of active compound are taken at frequent intervals, and for a sufficient duration to cover the terminal elimination part (e.g. Pre-dose, 1, 2, 3, 4, 5, 6, 7, 8, 10, 12, 24 (day 2), 36 (day 2), 48 (day 3), 60 (day 3), 72 (day 4) and 84 (day 4) hours post dose). Determination of the concentration of active compound is performed as described in Wilken et al., Diabetologia 43(51):A143, 2000. Derived pharmacokinetic parameteres are calculated from the concentration-time data for each individual subject by use of non-compartmental methods, using the commercially available software WinNonlin Version 2.1 (Pharsight, Cary, NC, USA). The terminal elimination rate constant is estimated by log-linear regression on the terminal log-linear part of the concentration-time curve, and used for calculating the elimination half-life.
The term "DPP-IV protected exendin-4 compound" as used herein means an exendin-4 compound which has been chemically modified to render said compound resistant to the plasma peptidase dipeptidyl aminopeptidase-4 (DPP-IV). The DPP-IV enzyme in plasma is known to be involved in the degradation of several peptide hormones, e.g. GLP-1, GLP-2, Exendin-4 etc. Thus, a considerable effort is directed to develop analogues and derivatives of the polypeptides susceptible to DPP-IV mediated hydrolysis in order to reduce the rate of degradation by DPP-IV. Resistance of a peptide to degradation by dipeptidyl aminopeptidase IV is determined by the following degradation assay :
Aliquots of the peptide (5 nmol) are incubated at 37 ºC with 1 µL of purified dipeptidyl aminopeptidase IV corresponding to an enzymatic activity of 5 mU for 10-180 minutes in 100 µL of 0.1 M triethylamine-HCl buffer, pH 7.4. Enzymatic reactions are terminated by the addition of 5 µL of 10% trifluoroacetic acid, and the peptide degradation products are separated and quantified using HPLC analysis. One method for performing this analysis is : The mixtures are applied onto a Vydac C18 widepore (30 nm pores, 5 µm particles) 250 x 4.6 mm column and eluted at a flow rate of 1 ml/min with linear stepwise gradients of acetonitrile in 0.1 % trifluoroacetic acid (0% acetonitrile for 3 min, 0-24% acetonitrile for 17 min, 24-48% acetonitrile for 1 min) according to Siegel et al., Regul. Pept. 1999;79:93-102 and Mentlein et al. Eur. J. Biochem. 1993;214:829-35. Peptides and their degradation products may be monitored by their absorbance at 220 nm (peptide bonds) or 280 nm (aromatic amino acids), and are quantified by integration of their peak areas related to those of standards. The rate of hydrolysis of a peptide by dipeptidyl aminopeptidase IV is estimated at incubation times which result in less than 10% of the peptide being hydrolysed.

The term "exendin-4 fusion protein" as used herein means a fusion protein between an exendin-4 compound and a second polypeptide, optionally via a spacer peptide. The second polypeptide is typically selected so as to confer stability of the fusion protein in plasma or to enhance bioavailability. Examples of the second polypeptide are human serum albumin (HSA), HSA fragments, IgG1, and Fc portion of IgG1. Further examples of exendin-4 fusion proteins are listed in WO 02/46227.

The term "immunomodulated exendin-4" as used herein means an exendin-4 compound which is a chemically modified exendin-4(1-39) having a reduced immune response in humans as compared to exendin-4(1-39). The method for assessing the immune response is to measure the concentration of antibodies reactive to the exendin-4 compound after 4 weeks of treatment of the patient.

The term "pharmaceutically acceptable" as used herein means suited for normal pharmaceutical applications, i.e. giving rise to no adverse events in patients etc.

### DETAILED DESCRIPTION OF THE INVENTION

A wide range of exendin-4 compounds have been disclosed in the art. Examples of exendin-4(1-39), insulinotropic fragments thereof, insulinotropic analogs thereof and insulinotropic derivatives thereof are described in WO 98/08871, WO 99/43706, US 5424286, WO 00/09666, WO 00/66629, WO 01 /04156 and WO 02/90388.

Thiazolidinediones are a class of compounds which comprises a thiazolidinedione functionality, and they have proven to be strong insulin sensitizers, i.e. to enhance the bodies responsiveness to the insulin present in plasma (see e.g. WO 97/41097). Through their interaction thiazolidinediones provides glyecaemic control via their insulin sensitization in the treatment of hyperglycaemia in patients with diabetes.

We have discovered that in the treatment of diabetes and of diabetes related diseases there is a synergistic effect of exendin-4 compounds and thiazolidinedione insulin sensitizers. A synergistic effect of two compounds is in terms of statistical analysis an effect which is greater than the additive effect. Animal models of diabetes and diabetes related diseases wherein combination treatment with exendin-4 compound and thiazolidinedione insulin sensitizers exhibit synergistic effect. This is an important finding as thiazolidinedione insulin senstizers have side effects well documented in clinical use for many years, but also because clinical tests indicate immunigenicity as being one side effect of exendin-4, a possible consequence of exendin-4 being a lizard peptide alien to the human immune system.

A synergistic effect of two compounds permits the dosages of these compounds in the combined treatment to be below the optimal dosages of the individual compounds in single-compound treatment. Thus, these suboptimal dosages of the individual compounds reduce side effects since lower dosages are needed for the same therapeutic effect in the combined treatment.

Accordingly, the present invention relates to methods for treatment of diabetes and diabetes related diseases selected from the group consisting of type 1 diabetes, type 2 diabetes, hyperglycemia, type 1.5 diabetes, latent autoimmune diabetes in adults, maturity onset diabetes, beta-cell apoptosis, hemochromatosis induced diabetes, impaired glucose tolerance, metabolic syndrome X, insulin resistance, cystic fibrosis related diabetes, polycystic ovarian syndrome, gestational diabetes, obesity, dyslipidemia, diabetic dyslipidemia, hyperlipidemia, hypertriglyceridemia, hyperlipoproteinemia, hypercholesterolemia, hypertension, essential hypertension, acute hypertensive emergency, arteriosclerosis, atherosclerosis, intermittent claudication (atherosclerosis oblitterens), cardiovascular disease, cardiomyopathy, cardiac hypertrophy, left ventricular hypertrophy, coronary artery disease, early coronary artery disease, heart insufficiency, exercise tolerance, chronic heart failure, mild chronic heart failure, arrhythmia, cardiac dysrythmia, syncopy, heart attack, myocardial infarction, Q-wave myocardial infarction, stroke, acute coronary syndrome, angina pectoris, unstable angina, cardiac bypass reocclusion, diastolic dysfunction, systolic dysfunction, non-Q-wave cardiac necrosis, catabolic changes after surgery, acute pancreatitis, irritable bowel syndrome, diabetic retinopathy, background retinopathy, preproliferative retinopathy, proliferative retinopathy, macular edema, cataracts, nephropathy, diabetic nephropathy, microalbuminuria, macroalbuminuria, neuropathy, diabetic neuropathy, distal symmetrical sensorimotor polyneuropathy, and diabetic autonomic neuropathy, which methods comprise administration of an effective amount of an exendin-4 compound and administration of an effective amount of a thiazolidinedione insulin sensitizer to a patient in need thereof.

The two compounds, i.e. the exendin-4 compound and the thiazolidinedione insulin sensitizer, may be co-administered or they may be administered separately as two medicaments. Furthermore, the first compound may be administered in a regimen, which additionally comprises treatment with the second compound. Hence, according to the present invention the only provision is that there must be overlapping periods of treatment with the exendin-4 compound and the thiazolidinedione insulin sensitizer.

In a second aspect the present invention relates to methods for increasing the number of beta-cells in a patient, increasing the size of beta-cells in a patient or stimulating beta-cell proliferation in a patient in need thereof, which method comprises administration of an effective amount of an exendin-4 compound and an effective amount of a thiazolidinedione insulin sensitizer to a patient in need thereof.

The two compounds may be co-administered or they may be administered separately as two medicaments. Furthermore, the first compound may be administered in a regimen, which additionally comprises treatment with the second compound. Hence, according to the present invention the only provision is that there must be overlapping periods of treatment with the exendin-4 compound and the thiazolidinedione insulin sensitizer.

In a third aspect the present invention relates to pharmaceutical compositions comprising an exendin-4 compound, a thiazolidinedione insulin sensitizer and a pharmaceutically acceptable preservative.

In one embodiment the exendin-4 compound is selected from the group consisting of exendin-4, an exendin-4 analogue, an exendin-4 derivative, an exendin-4 fusion protein, a DPPIV protected exendin-4, and an immunomodulated exendin-4 .

In another embodiment the exendin-4 compound has a binding affinity towards the GLP-1 receptor which is at least ten fold higher than that of GLP-1 (7-37), at least 100 fold higher than that of GLP-1 (7-37), at least the same affinity towards the GLP-1 receptor as that of exendin-4(1-39), preferable at least twice the affinity as that of exendin-4(1-39). The potency of an exendin-4 compound is determined by calculating the EC₅₀ value from the dose-response curve as described below.
Baby hamster kidney (BHK) cells expressing the cloned human GLP-1 receptor (BHK-467-12A) were grown in DMEM media with the addition of 100 IU/mL penicillin, 100 µL/mL streptomycin, 10% fetal calf serum and 1 mg/mL Geneticin G-418 (Life Technologies). Plasma membranes were prepared by homogenisation in buffer (10 mM Tris-HCl, 30 mM NaCl and 1 mM dithiothreitol, pH 7.4, containing, in addition, 5 mg/L leupeptin (Sigma, St. Louis, MO, USA), 5 mg/L pepstatin (Sigma), 100 mg/L bacitracin (Sigma), and 16 mg/L aprotinin (Calbiochem-Novabiochem, La Jolla, CA). The homogenate was centrifuged on top of a layer of 41 w/v% sucrose. The white band between the two layers was diluted in buffer and centrifuged. Plasma membranes were stored at -80°C until used.
The functional receptor assay was carried out by measuring cAMP as a response to stimulation by the exendin-4 compound. Incubation were carried out in 96-well microtiter plates in a total volume of 140 µL and with the following final concentrations: 50 mM Tris-HCl, 1 mM EGTA, 1.5 mM MgSO₄, 1.7 mM ATP, 20 mM GTP, 2 mM 3-isobutyl-1-methylxanthine (IBMX), 0.01 % Tween-20, pH 7.4. Compounds to be tested for agonist activity were dissolved and diluted in buffer. GTP was freshly prepared for each experiment : 2.5 µg of membrane was added to each well and the mixture was incubated for 90 min at room temperature in the dark with shaking. The reaction was stopped by the addition of 25 µL of 0.5 M HCl. Formed cAMP was measured by a scintillation proximity assay (RPA 542, Amersham, UK). Dose-response curves were plotted for the individual compounds and EC₅₀ values calculated using GraphPad Prism software.

In yet another embodiment the exendin-4 compound is (Lys)₆-exendin-4. In yet another embodiment the exendin-4 compound is ZP-1 0, i.e. Ser³⁸,Lys³⁹, ^{40, 41, 42, 43, 44}-Exendin-4(1-39)-amide.

In yet another embodiment the exendin-4 compound is a fusion protein which comprises exendin-4, an analogue thereof or a derivative of any one of the foregoing, coupled optionally via a spacer to a stabilizing peptide which confers an increased half-life in human serum to said fusion protein as compared to the fusion protein devoid of said spacer and stabilizing peptide.

In yet another embodiment the spacer is selected from the group consisting of a peptide so that said fusion protein can be directly produced by recombinant DNA techniques, a peptide connected in either end in a way that said fusion protein cannot be produced directly by recombinant DNA techniques, a peptide comprising two amino acid residues and a non-peptide moiety.

In a further embodiment the exendin-4 compound comprises a sequence of 25 amino acids which are identical to a sequence of 25 amino acids in human serum albumin.

In a further embodiment the exendin-4 compound comprises a polyethylene glycol moiety. In a further embodiment the exendin-4 compound comprises a polyethylene glycol moiety which has a molar weight from 500 Dalton to 20000 Dalton, preferably from 5000 Dalton to 12000 Dalton. In a further embodiment the exendin-4 compound comprises two polyethylene glycol moieties.

In a further embodiment the exendin-4 compound is an acylated peptide. In a further embodiment the exendin-4 compound comprises a C₆-C₁₈ straight acyl moiety, preferable a C₈- or C₁₆-acyl moiety.

In a further embodiment the exendin-4 compound is acylated on the epsilon amino group of a lysine residue. In a further embodiment the exendin-4 compound is acylated on a glutamic acid residue.

In a further embodiment the immunomodulated exendin-4 has an immune response in humans which is less than that of exendin-4(1-39), preferable less than 20% of the response of exendin-4(1-39), said immune response being measured as the concentration of antibodies reactive to the exendin-4 compound after 4 weeks of treatment of the patient.

Exendin-4 compounds may be synthesized by procedures well known in the art. Biologically encoded peptides may be produced by recombinant DNA techniques, and subsequently be chemically modified if necessary. Exendin-4(1-39), analogs thereof as well as smaller fusion proteins may by produced by chemical synthesis by e.g. classical Merrifield-type solid phase synthesis. Introduction of chemical modifications are also well described in the art (see. e.g. WO 98/08871, WO 99/43706, US 5424286, WO 00/09666, WO 00/66629, WO 01/04156, WO 02/46227 and WO 02/90388).

In a further embodiment the thiazolidinedione insulin sensitizer is selected from the group consisting of troglitazone, ciglitazone, pioglitazone, rosiglitazone, isaglitazone, darglitazone, englitazone, CS-011/CI-1037, T174, and TZD 300512, 5-[[4-[3-methyl-4-oxo-3,4-dihydro-2-quinazolinyl]methoxy]phenyl-methyl]thiazolidine-2,4-dione or a salt thereof.

In a further embodiment the exendin-4 compound is exendin-4(1-39) and the thiazolidinedione insulin sensitizer is troglitazone, pioglitazone, 5-[[4-[3-methyl-4-oxo-3,4-dihydro-2-quinazolinyl]methoxy]phenyl-methyl]thiazolidine-2,4-dione, or a salt thereof.

In a further embodiment the exendin-4 compound is administered in a regimen which additionally comprises administration of said thiazolidinedione insulin sensitizer.

In a further embodiment the exendin-4 compound and said thiazolidinedione insulin sensitizer are co-administered.

In a further embodiment the exendin-4 compound is a parenteral medicament.

In a further embodiment the thiazolidinedione insulin sensitizer is an oral medicament.

In a further embodiment the exendin-4 compound and said thiazolidinedione insulin sensitizer are administrered in suboptimal dosages.

In a further embodiment the dosage of exendin-4 compound is from 1 µg/day to 50µg/day. In a further embodiment the dosage of exendin-4 compound is from 0.1 µg/day to 5µg/day. In a further embodiment the dosage of exendin-4 compound is from 2µg/day to 20µg/day.

In a further embodiment the dosage of thiazolidinedione insulin sensitizer is from 0.01 mg/day to 10 mg/day, preferably from 0.1 mg/day to 3 mg/day, most preferable less than 2 mg/day.

In a further embodiment the exendin-4 compound and said thiazolidinedione insulin sensitizer are administered in amounts and for a sufficient time to produce a synergistic effect.

The patient is preferably a mammal, more preferable a human.

In another aspect the invention relates to a pharmaceutical composition comprising an exendin-4 compound, a thiazolidinedione and a pharmaceutically acceptable preservative.

In one embodiment the pharmaceutical composition comprising an exendin-4 compound, a thiazolidinedione and a pharmaceutically acceptable preservative further comprises a buffer and an isotonicity agent.

In another embodiment the present invention relates to a pharmaceutical composition comprising an exendin-4 compound, a thiazolidinedione and a pharmaceutically acceptable preservative, wherein said exendin-4 compound is selected from exendin-4, an exendin-4 analogue, an exendin-4 derivative, an exendin-4 fusion protein, a DPPIV protected exendin-4, and an immunomodulated exendin-4.

In another embodiment the present invention relates to a pharmaceutical composition comprising an exendin-4 compound, a thiazolidinedione and a pharmaceutically acceptable preservative, wherein said thiazolidinedione insulin sensitizer is selected from the group consisting of troglitazone, ciglitazone, pioglitazone, rosiglitazone, isaglitazone, darglitazone, englitazone, CS-011/Cl-1037, T174, and TZD 300512, 5-[[4-[3-methyl-4-oxo-3,4-dihydro-2-quinazolinyl]methoxy]phenyl-methyl]thiazolidine-2,4-dione or a salt thereof.

In another embodiment the present invention relates to a pharmaceutical composition comprising an exendin-4 compound, a thiazolidinedione and a pharmaceutically acceptable preservative, wherein the concentration of said exendin-4 compound is from 0.005mg/ml to 0.5mg/ml and the concentration of said thiazolidinedione insulin sensitizer is from 0.5mg/ml to 20mg/ml.

In another embodiment the present invention relates to a pharmaceutical composition comprising an exendin-4 compound, a thiazolidinedione and a pharmaceutically acceptable preservative, wherein the concentration of said exendin-4 compound is from 0.01 mg/ml to 0.1 mg/ml and the concentration of said thiazolidinedione insulin sensitizer is from 1 mg/ml to 10mg/ml.

In another embodiment the present invention relates to the use of a pharmaceutical composition comprising an exendin-4 compound, a thiazolidinedione and a pharmaceutically acceptable preservative for the treatment of one or more diseases, conditions or disorders selected from the group consisting of type 1 diabetes, type 2 diabetes, hyperglycemia, type 1.5 diabetes, latent autoimmune diabetes in adults, maturity onset diabetes, beta-cell apoptosis, hemochromatosis induced diabetes, impaired glucose tolerance, metabolic syndrome X, insulin resistance, cystic fibrosis related diabetes, polycystic ovarian syndrome, gestational diabetes, obesity, dyslipidemia, diabetic dyslipidemia, hyperlipidemia, hypertriglyceridemia, hyperlipoproteinemia, hypercholesterolemia, hypertension, essential hypertension, acute hypertensive emergency, arteriosclerosis, atherosclerosis, intermittent claudication (atherosclerosis oblitterens), cardiovascular disease, cardiomyopathy, cardiac hypertrophy, left ventricular hypertrophy, coronary artery disease, early coronary artery disease, heart insufficiency, exercise tolerance, chronic heart failure, mild chronic heart failure, arrhythmia, cardiac dysrythmia, syncopy, heart attack, myocardial infarction, Q-wave myocardial infarction, stroke, acute coronary syndrome, angina pectoris, unstable angina, cardiac bypass reocclusion, diastolic dysfunction, systolic dysfunction, non-Q-wave cardiac necrosis, catabolic changes after surgery, acute pancreatitis, irritable bowel syndrome, diabetic retinopathy, background retinopathy, preproliferative retinopathy, proliferative retinopathy, macular edema, cataracts, nephropathy, diabetic nephropathy, microalbuminuria, macroalbuminuria, neuropathy, diabetic neuropathy, distal symmetrical sensorimotor polyneuropathy, and diabetic autonomic neuropathy.

In another embodiment the present invention relates to the use of a pharmaceutical composition comprising an exendin-4 compound, a thiazolidinedione and a pharmaceutically acceptable preservative for increasing the number of beta-cells in a patient, increasing the size of beta-cells in a patient or stimulating beta-cell proliferation in a patient in need thereof

The route of administration may be any route, which effectively transports the active compound to the appropriate or desired site of action, such as oral, sublingual, nasal, tracheal, buccal, pulmonal, transdermal or parenteral.

Pharmaceutical compositions (or medicaments) containing an exendin-4 compound, such as exendin-4(1-39), may be administered parenterally to patients in need of such a treatment. Parenteral administration may be performed by subcutaneous, intramuscular or intravenous injection by means of a syringe, optionally a pen-like syringe. Alternatively, parenteral administration can be performed by means of an infusion pump. A further option is a composition which may be a powder or a liquid for the administration of exendin-4 compounds in the form of a nasal or pulmonal spray. As a still further option, the exendin-4 compound can also be administered transdermally, e.g. from a patch, optionally a iontophoretic patch, or transmucosally, e.g. bucally. The above-mentioned possible ways to administer exendin-4 compounds are not considered as limiting the scope of the invention.

Pharmaceutical compositions containing exendin-4 compounds, such as exendin-4(1-39), may be prepared by conventional techniques, e.g. as described in Remington's *Pharmaceutical Sciences,* 1985 or in Remington: *The Science and Practice of Pharmacy,* 19^{th} edition, 1995, and as described in WO 99/43708 and WO 00/41546

Thus, the injectable compositions of exendin-4 compounds can be prepared using the conventional techniques of the pharmaceutical industry which involves dissolving and mixing the ingredients as appropriate to give the desired end product.

According to one procedure, e.g. exendin-4(1-39) is dissolved in an amount of water which is somewhat less than the final volume of the composition to be prepared. An isotonic agent, a preservative and a buffer are added as required and the pH value of the solution is adjusted - if necessary - using an acid, e.g. hydrochloric acid, or a base, e.g. aqueous sodium hydroxide as needed. Finally, the volume of the solution is adjusted with water to give the desired concentration of the ingredients.

Examples of isotonicity agents are sodium chloride, mannitol, glycerol and dimethyl sulfone.

Examples of preservatives are phenol, m-cresol, methyl p-hydroxybenzoate and benzyl alcohol.

Examples of suitable buffers are sodium acetate, sodium phosphate, TRIS, glycine, and glycylglycine

Further to the above-mentioned components, solutions containing an exendin-4 compound may also contain a surfactant and/or stabilizer in order to improve the solubility and/or the stability of the peptide.

According to one embodiment of the present invention, the exendin-4 compound is provided in the form of a composition suitable for administration by injection. Such a composition can either be an injectable solution ready for use or it can be an amount of a solid composition, e.g. a lyophilised product, which has to be dissolved in a solvent before it can be injected. The injectable solution preferably contains not less than about 0.1 mg/ml, typically from 0.1 mg/ml to 5 mg/ml, such as from 0.5 mg/ml to 5 mg/ml of exendin-4 compound.

Exendin-4 compounds such as exendin-4(1-39) can be used in the treatment of various diseases. The optimal dose level for any patient (effective dosage) will depend on the disease to be treated and on a variety of factors including the efficacy of the specific exendin-4 compound employed, the age, body weight, physical activity, and diet of the patient, on a possible combination with other drugs, and on the severity of the case.

Throughout the present application an effective dosage is defined as a dosage which is sufficient in order for the treatment of the patient to be effective. A suboptimal dosage of a pharmaceutically active compound is defined as a dosage which is below the optimal dosage for that compound when used in single-compound therapy.

Pharmaceutical compositions (or medicaments) containing thiazolidinedione insulin sensitizers, such as 5-[[4-[3-methyl-4-oxo-3,4-dihydro-2-quinazolinyl]methoxy]phenylmethyl]thiazolidine-2,4-dione, may be administered by suitable dosage forms such as oral, nasal, pulmonal, buccal or transdermal to patients in need of such a treatment. The preferred route of administration of non-thiazolidinedione PPAR ligands is orally. Pharmaceutical compositions containing non-thiazolidinedione PPAR ligands may be prepared by conventional techniques, e.g. as described in Remington: The Science and Practice of Pharmacy, 19th Edition, Gennaro, Ed., Mack Publishing Co., Easton, PA, 1995.

Typical compositions of e.g. 5-[[4-[3-methyl-4-oxo-3,4-dihydro-2-quinazolinyl]methoxy] phenyl-methyl]thiazolidine-2,4-dione include a crystalline compound of the present invention associated with a pharmaceutically acceptable excipient, which may be a carrier or a diluent or be diluted by a carrier, or enclosed within a carrier, which can be in the form of a capsule, sachet, paper or other container. In making the compositions, conventional techniques for the preparation of pharmaceutical compositions may be used. For example, the active compound will usually be mixed with a carrier, or diluted by a carrier, or enclosed within a carrier, which may be in the form of a ampoule, capsule, sachet, paper, or other container. When the carrier serves as a diluent, it may be solid, semi-solid, or liquid material, which acts as a vehicle, excipient, or medium for the active compound. The active compound can be adsorbed on a granular solid container for example in a sachet. Some examples of suitable carriers are water, salt solutions, alcohol's, polyethylene glycol's, polyhydroxyethoxylated castor oil, peanut oil, olive oil, gelatine, lactose, terra alba, sucrose, cyclodextrin, amylose, magnesium stearate, talc, gelatine, agar, pectin, acacia, stearic acid or lower alkyl ethers of cellulose, silicic acid, fatty acids, fatty acid amines, fatty acid monoglycerides and diglycerides, pentaerythritol fatty acid esters, polyoxyethylene, hydroxymethylcellulose and polyvinylpyrrolidone. Similarly, the carrier or diluent may include any sustained release material known in the art, such as glyceryl monostearate or glyceryl distearate, alone or mixed with a wax. The formulations may also include wetting agents, emulsifying and suspending agents, preserving agents, sweetening agents or flavouring agents. The formulations of the invention may be formulated so as to provide quick, sustained, or delayed release of the active ingredient after administration to the patient by employing procedures well known in the art.

The pharmaceutical compositions can be sterilized and mixed, if desired, with auxiliary agents, emulsifiers, salt for influencing osmotic pressure, buffers and/or colouring substances and the like, which do not deleteriously react with the active compound.

If a solid carrier is used for oral administration, the preparation may be tabletted, placed in a hard gelatine capsule in powder or pellet form or it can be in the form of a troche or lozenge. If a liquid carrier is used, the preparation may be in the form of a syrup, emulsion, soft gelatine capsule or sterile injectable liquid such as an aqueous or non-aqueous liquid suspension or solution.

For nasal administration, the preparation may contain the compound of the present invention dissolved or suspended in a liquid carrier, in particular an aqueous carrier, for aerosol application. The carrier may contain additives such as solubilizing agents, e.g. propylene glycol, surfactants, absorption enhancers such as lecithin (phosphatidylcholine) or cyclodextrin, or preservatives such as parabenes.

For parenteral application, particularly suitable are injectable solutions or suspensions, preferably aqueous solutions with the active compound dissolved in polyhydroxylated castor oil.

Tablets, dragees, or capsules having talc and/or a carbohydrate carrier or binder or the like are particularly suitable for oral application. Preferable carriers for tablets, dragees, or capsules include lactose, cornstarch, and/or potato starch. A syrup or elixir can be used in cases where a sweetened vehicle can be employed.

A typical tablet of a thiazolidinedione insulin sensitizer, e.g. 5-[[4-[3-methyl-4-oxo-3,4-dihydro-2-quinazolinyl]methoxy]phenyl-methyl]thiazolidine-2,4-dione, which may be prepared by conventional tabletting techniques, may contain:

| Core: | |
|---|---|
| Active compound | 5 mg |
| Colloidal silicon dioxide (Aerosil) | 1.5 mg |
| Cellulose, microcryst. (Avicel) | 70 mg |
| Modified cellulose gum (Ac-Di-Sol) | 7.5 mg |
| Magnesium stearate | Ad. |

| Coating: | |
|---|---|
| HPMC approx. | 9 mg |
| *Mywacett 9-40 T approx. | 0.9 mg |

| | |
|---|---|
| *Acylated monoglyceride used as plasticizer for film coating. | |

The thiazolidinedione insulin sensitizers are effective over a wide dosage range. For example, in the treatment of adult humans, dosages from 0.01 mg/day to 10 mg/day, preferably from 0.1 mg/day to 3 mg/day may be used. A most preferable dosage is less than 2 mg/day. In choosing a regimen for patients it may frequently be necessary to begin with a dosage of from about 2 to about 10 mg per day and when the condition is under control to reduce the dosage as low as from about 0.01 to about 3 mg per day. The exact dosage will depend upon the mode of administration, on the therapy desired, the administration form, the subject to be treated and the body weight of the subject to be treated.

Generally, the thiazolidinedione insulin sensitizers of the present invention are dispensed in unit dosage form comprising from about 0.01 to about 10 mg of active ingredient together with a pharmaceutically acceptable carrier per unit dosage.

Usually, dosage forms suitable for oral, nasal, pulmonary or transdermal administration comprise from about 0.01 mg to about 10 mg, preferably from about 0.1 mg to about 3 mg of the compound of the invention admixed with a pharmaceutically acceptable carrier or diluent.

Irrespective of the dosage forms for the exendin-4 compound and for the thiazolidinedione insulin sensitizer, they may advantageously be supplied as a kit for treatment of diabetes or diabetes related disease selected from the group consisting of type 1 diabetes, type 2 diabetes, hyperglycemia, type 1.5 diabetes, latent autoimmune diabetes in adults, maturity onset diabetes, beta-cell apoptosis, hemochromatosis induced diabetes, impaired glucose tolerance, metabolic syndrome X, insulin resistance, cystic fibrosis related diabetes, polycystic ovarian syndrome, gestational diabetes, obesity, dyslipidemia, diabetic dyslipidemia, hyperlipidemia, hypertriglyceridemia, hyperlipoproteinemia, hypercholesterolemia, hypertension, essential hypertension, acute hypertensive emergency, arteriosclerosis, atherosclerosis, intermittent claudication (atherosclerosis oblitterens), cardiovascular disease, cardiomyopathy, cardiac hypertrophy, left ventricular hypertrophy, coronary artery disease, early coronary artery disease, heart insufficiency, exercise tolerance, chronic heart failure, mild chronic heart failure, arrhythmia, cardiac dysrythmia, syncopy, heart attack, myocardial infarction, Q-wave myocardial infarction, stroke, acute coronary syndrome, angina pectoris, unstable angina, cardiac bypass reocclusion, diastolic dysfunction, systolic dysfunction, non-Q-wave cardiac necrosis, catabolic changes after surgery, acute pancreatitis, irritable bowel syndrome, diabetic retinopathy, background retinopathy, preproliferative retinopathy, proliferative retinopathy, macular edema, cataracts, nephropathy, diabetic nephropathy, microalbuminuria, macroalbuminuria, neuropathy, diabetic neuropathy, distal symmetrical sensorimotor polyneuropathy, and diabetic autonomic neuropathy.

Irrespective of the dosage forms for the exendin-4 compound and for the thiazolidinedione insulin sensitizer, they may advantageously be supplied as a kit for increasing the number of beta-cells in a patient, increasing the size of beta-cells in a patient or stimulating beta-cell proliferation in a patient in need thereof, which method comprises administration of an effective amount of an exendin-4 compound and an effective amount of a thiazolidinedione insulin sensitizer to a patient in need thereof.

The kit may contain a single dosage form or it may contain two dosage forms, i.e. one for each compound to be administered.

The exendin-4 compound and the thiazolidinedione insulin sensitizer may also be in a single pharmaceutical composition, preferable a parental composition, as described above.

The combined treatment with an exendin-4 compound and a thiazolidinedione insulin sensitizer may also be combined with a third or more further pharmacologically active substances, e.g. selected from antidiabetic agents, antiobesity agents, appetite regulating agents, antihypertensive agents, agents for the treatment and/or prevention of complications resulting from or associated with diabetes and agents for the treatment and/or prevention of complications and disorders resulting from or associated with obesity. Examples of these pharmacologically active substances are : Insulin, GLP-1 agonists, sulphonylureas, biguanides, meglitinides, glucosidase inhibitors, glucagon antagonists, DPP-IV (dipeptidyl peptidase-IV) inhibitors, inhibitors of hepatic enzymes involved in stimulation of gluconeogenesis and/or glycogenolysis, glucose uptake modulators, compounds modifying the lipid metabolism such as antihyperlipidemic agents and antilipidemic agents as HMG CoA inhibitors (statins), compounds lowering food intake, RXR agonists and agents acting on the ATP-dependent potassium channel of the β-cells; Cholestyramine, colestipol, clofibrate, gemfibrozil, lovastatin, pravastatin, simvastatin, probucol, dextrothyroxine; β-blockers such as alprenolol, atenolol, timolol, pindolol, propranolol and metoprolol, ACE (angiotensin converting enzyme) inhibitors such as alatriopril, benazepril, captopril, enalapril, fosinopril, lisinopril, quinapril and ramipril, calcium channel blockers such as nifedipine, felodipine, nicardipine, isradipine, nimodipine, diltiazem and verapamil, and α-blockers such as doxazosin, urapidil, prazosin and terazosin; CART (cocaine amphetamine regulated transcript) agonists, NPY (neuropeptide Y) antagonists, MC4 (melanocortin 4) agonists, orexin antagonists, TNF (tumor necrosis factor) agonists, CRF (corticotropin releasing factor) agonists, CRF BP (corticotropin releasing factor binding protein) antagonists, urocortin agonists, β3 agonists, MSH (melanocyte-stimulating hormone) agonists, MCH (melanocyte-concentrating hormone) antagonists, CCK (cholecystokinin) agonists, serotonin re-uptake inhibitors, serotonin and noradrenaline re-uptake inhibitors, mixed serotonin and noradrenergic compounds, 5HT (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA agonists (bromocriptin, doprexin), lipase/amylase inhibitors, RXR (retinoid X receptor) modulators, TRβ agonists; histamine H3 antagonists.

It should be understood that any suitable combination of the compounds according to the invention with one or more of the above-mentioned compounds and optionally one or more further pharmacologically active substances are considered to be within the scope of the present invention.

### EXPERIMENTAL

Synergistic effect of combining thiazolidinedione insulin sensitizer and exendin-4(1-39) on glucose and HbA_{1c} (glycosylated hemoglobin) in the male ZDF rat.

### Study design:

Male ZDF rats were used in the study. Before treatment start, measurements of glucose and HbA_{1c} were performed. All animals were overtly diabetic at the beginning of the study. Animals were allocated into the following 4 treatment groups:
- Group 1:: Vehicle-1 + Vehicle-2
- Group 2:: thiazolidinedione insulin sensitizer, x mg/kg + Vehicle-2 (n=10)
- Group 3:: Vehicle-1 + exendin-4(1-39), xx µg/kg
- Group 4:: thiazolidinedione insulin sensitizer , x mg/kg + exendin-4(1-39), xx µg/kg

Thiazolidinedione insulin sensitizer and Vehicle-1 were administered by oral gavage once daily at approx. 07:30. Exendin-4(1-39) and Vehicle-2 were administered subcutaneously twice daily at approx. 07:30 and 14:30.

After four weeks treatment, HbA_{1c} was measured and 24-hour glucose profiles were assessed.

### Results:

The findings of group 1, 2, 3 and 4 are listed. Delta HbA_{1c} values referring to the numerical difference between the measurement after treatment and the measurement before treatment are calculated. Glucose_{24hAUC} refers to the total area under the glucose concentration curve during the 24-hour period. A two-way analysis of variance are performed for each parameter and the significance of the interaction term evaluated.

A highly significant interaction terms demonstrate that four weeks combination treatment with thiazolidinedione insulin sensitizer (x mg/kg, once daily) and Exendin-4(1-39) (xx µg/kg, twice daily) has synergistic (greater than additive) effects on HbA_{1c} and 24-hour glucose profiles in overtly diabetic ZDF rats.

## Claims

1. Use of an exendin-4 compound and a thiazolidinedione insulin sensitizer for the preparation of one or more medicaments for the treatment or prevention of diabetes or a diabetes related disease in a patient in need thereof.

2. The use according to claim 1, wherein said diabetes or diabetes related disease is selected from the group consisting of type 1 diabetes, type 2 diabetes, hyperglycemia, type 1.5 diabetes, latent autoimmune diabetes in adults, maturity onset diabetes, beta-cell apoptosis, hemochromatosis induced diabetes, impaired glucose tolerance, metabolic syndrome X, insulin resistance, cystic fibrosis related diabetes, polycystic ovarian syndrome, and gestational diabetes.

3. The use according to any one of claims 1-2, wherein said exendin-4 compound is selected from the group consisting of exendin-4, an exendin-4 analogue, an exendin-4 derivative, an exendin-4 fusion protein, a DPPIV protected exendin-4, and an immunomodulated exendin-4.

4. The use according to any one of claims 1-3, wherein said exendin-4 compound is a fusion protein which comprises exendin-4, an analogue thereof or a derivative of any one of the foregoing, coupled optionally via a spacer to a stabilizing peptide which confers an increased half-life in human serum to said fusion protein as compared to the fusion protein devoid of said spacer and stabilizing peptide.

5. The use according to any one of claims 3-4, wherein said exendin-4 compound comprises a polyethylene glycol (PEG) moiety.

6. The use according to any one of claim 3-5, wherein said immunomodulated exendin-4 has an immune response in humans which is less than that of exendin-4(1-39), preferable less than 20% of the response of exendin-4(1-39), said immune response being measured as the concentration of antibodies reactive to the exendin-4 compound after 4 weeks of treatment of the patient.

7. The use according to any one of claims 1-6, wherein said thiazolidinedione insulin sensitizer is selected from the group consisting of troglitazone, ciglitazone, pioglitazone, rosiglitazone, isaglitazone, darglitazone, englitazone, CS-011/Cl-1037, T174, and TZD 300512, 5-[[4-[3-methyl-4-oxo-3,4-dihydro-2-quinazolinyl]methoxy]phenyl-methyl]thiazolidine-2,4-dione or a salt thereof.

8. The use according to any one of claims 1-3 and 7, wherein the exendin-4 compound is exendin-4(1-39) and the thiazolidinedione insulin sensitizer is troglitazone, pioglitazone, 5-[[4-[3-methyl-4-oxo-3,4-dihydro-2-quinazolinyl]methoxy]phenyl-methyl]thiazolidine-2,4-dione, or a salt thereof.

9. The use according to any one of claims 1-8, wherein said exendin-4 compound is administered in a regimen which additionally comprises administration of said thiazolidinedione insulin sensitizer.

10. The use according to any one of claims 1-9, wherein said exendin-4 compound and said thiazolidinedione insulin sensitizer are co-administered.

11. The use according to any one of claims 1-10, wherein said exendin-4 compound is a parenteral medicament.

12. The use according to any one of claims 1-11, wherein the dosage of exendin-4 compound is from 1 µg/day to 50 µg/day.

13. The use according to any one of claims 1-12, wherein the dosage of thiazolidinedione insulin sensitizer is from 0.01 mg/day to 10 mg/day, preferably from 0.1 mg/day to 3 mg/day, most preferable less than 2 mg/day.

14. The use according to any one of claims 1-13, wherein said exendin-4 compound and said thiazolidinedione insulin sensitizer are administered in amounts and for a sufficient time to produce a synergistic effect.

15. A pharmaceutical composition comprising an exendin-4 compound, a thiazolidinedione and a pharmaceutically acceptable preservative.

16. The pharmaceutical composition according to claim 15, further comprising a buffer and an isotonicity agent.

17. The pharmaceutical composition according to any one of claims 15-16, wherein said exendin-4 compound is selected from the compounds listed in any one of claims 3-6.

18. The pharmaceutical composition according to any one of claims 15-17, wherein said thiazolidinedione insulin sensitizer is selected from the group consisting of troglitazone, ciglitazone, pioglitazone, rosiglitazone, isaglitazone, darglitazone, englitazone, CS-011/Cl-1037, T174, and TZD 300512, 5-[[4-[3-methyl-4-oxo-3,4-dihydro-2-quinazolinyl]methoxy]phenylmethyl]thiazolidine-2,4-dione or a salt thereof.

19. The pharmaceutical composition according to any one of claims 15-18, wherein the concentration of said exendin-4 compound is from 0.005mg/ml to 0.5mg/ml and the concentration of said thiazolidinedione insulin sensitizer is from 0.5mg/ml to 20mg/ml.

20. The pharmaceutical composition according to any one of claims 15-19, wherein the concentration of said exendin-4 compound is from 0.01 mg/ml to 0.1 mg/ml and the concentration of said thiazolidinedione insulin sensitizer is from 1 mg/ml to 10mg/ml.
